(19) **Europäisches Patentamt / European Patent Office / Office européen des brevets**

(11) **EP 1 455 845 B1**

(12) **EUROPEAN PATENT SPECIFICATION**

(45) Date of publication and mention
of the grant of the patent:
**07.03.2007 Bulletin 2007/10**

(21) Application number: **02793247.4**

(22) Date of filing: **18.12.2002**

(51) Int Cl.:
**A61L 15/24** *(2006.01)*    **A61L 15/42** *(2006.01)*

(86) International application number:
**PCT/GB2002/005763**

(87) International publication number:
**WO 2003/053483 (03.07.2003 Gazette 2003/27)**

(54) **AN ABSORBENT HYGIENE PRODUCT**

EIN ABSORBIERENDES HYGIENEPRODUKT

PRODUIT D'HYGIENE ABSORBANT

(84) Designated Contracting States:
**AT BE BG CH CY CZ DE DK EE ES FI FR GB GR IE IT LI LU MC NL PT SE SI SK TR**

(30) Priority: **20.12.2001 GB 0130461**

(43) Date of publication of application:
**15.09.2004 Bulletin 2004/38**

(73) Proprietor: **Freundenberg Nonwovens LP**
**Greetland**
**Halifax**
**HX4 8NJ (GB)**

(72) Inventors:
• **GENTILCORE, Giovanni**
**Swindon,**
**Wiltshire SN1 4HU (GB)**

• **COOK, John Anthony**
**Faringdon,**
**Oxfordshire SN7 8SJ (GB)**
• **BEKKAOUI, Ali**
**Redhouse-Swindon-SN25 2DG (GB)**

(74) Representative: **Belcher, Simon James**
**Urquhart-Dykes & Lord LLP**
**Tower North Central**
**Merrion Way**
**Leeds LS2 8PA (GB)**

(56) References cited:
**WO-A-00/01468        WO-A-93/01622**
**WO-A-98/58108        US-A- 5 304 404**
**US-A- 5 382 703**

**Description**

This invention relates to an absorbent hygiene product and to a method of making an absorbent hygiene product.

Absorbent articles are known which contain a core of an absorbent material and can be used to absorb and contain body exudates. They can be used for personal applications for example as diapers, sanitary napkins, tampons, incontinent pads, training pants. They can be used for medical applications such as bandages and wound dressings. They can be used for cleaning applications for example as wipes. Such articles are often disposable in that they are not generally intended to be laundered or otherwise restored for reuse.

Suitable absorbent material for the core of an absorbent article will depend on the nature of the fluids which are to be absorbed. Examples of suitable materials include those disclosed in US-4950264, US-4610678, US-4834735, US-4673402 and US-4888231.

Absorbent articles of this kind often include one or more top sheets. A top sheet can serve to reduce leakage of fluid from the absorbent material in the core of the article. It can protect the absorbent material from damage. It can provide a surface which can improve comfort for the user, especially when it provides the surface which contacts the user's skin, for example by being soft to the touch and smooth. It can usefully promote rapid wicking of fluids in the plane of the sheet so that they are distributed across the core of absorbent material. A top sheet should be porous so that fluids can pass through it. The sheet can be made from fibres, especially as a non-woven fabric in which the pores are provided by spaces between fibres. The sheet should be wettable at least on one surface by the fluids which are to be absorbed by the article. The sheet can contain a number of sheet components, of which at least one should be wettable on at least one surface. These characteristics should be stable so that they do not deteriorate significantly when the article is stored prior to use. They should also be stable so that the characteristics are not changed significantly on exposure to fluid. In this way, the article can withstand repeated exposures to fluid.

It is known to treat sheets formed from polyolefins or other hydrophobic polymers with surface treatment agents to render the fabrics wettable. The treatment of fabrics in this way with surfactants is disclosed in US-5540984 and EP-A-669420, and the treatment of fabrics with polyvinyl acetate is disclosed in WO-00/37736. The treatment of a fabric with a hydrophilic material should be such that interactions between the fabric and the material are able to withstand processing of the fabric during manufacture of the absorbent article, and also to withstand exposure of the fabric to a fluid. For example, some surface treatment agents can dissolve, at least partially, in the fluid which the article is intended to absorb. As well as tending to affect the wettability of the fabric, dissolution of any surface treatment agent in the absorbed fluid can change the surface tension of the fluid, potentially reducing the ability of the article to absorb and retain fluid.

US-5382703 discloses vinyl monomer compounds which can be grafted to polyolefin non-woven fabrics in a reaction which is initiated by electron beam irradiation, The treated fabrics can be used in disposable absorbent products.

The present invention provides an absorbent hygiene product in which a porous sheet surface is provided by a hydrophobic polymeric material whose surface has been modified by a graft copolymerisation reaction between the surface and a vinyl monomer having a group which exhibits or can be changed so that it exhibits hydrophilic properties.

Accordingly, in one aspect, the present invention provides an absorbent hygiene product which comprises a core of an absorbent material and a top sheet through which fluid to be absorbed passes for absorption by the absorbent material, in which the top sheet is porous and comprises a sheet having surfaces provided by a hydrophobic polymeric material which have been modified by a graft copolymerisation reaction between the surfaces and a vinyl monomer which is an ethylenically unsaturated acid or an ester thereof so that the monomer exhibits or can be changed so that it exhibits hydrophilic properties, and in which the product is made by a process which involves exposure of the sheet to ultraviolet radiation while impregnated with a solution of the vinyl monomer.

The hygiene product of the present invention has the advantage that the treatment of the hydrophobic polymer of the top sheet results in it being wettable by aqueous solutions (including water). The wettability of the sheet can promote rapid wicking of aqueous solutions across the sheet. The fact that the monomer is grafted to surfaces of the hydrophobic polymer sheet means that the wettable characteristics of the sheet after the treatment are stable. The tendency of the surface tension of fluids to reduce when the sheet is exposed to them is reduced compared with sheets which are treated by application of a surfactant.

Furthermore, the top sheet in the hygiene product of the present invention can have a degree of creep resistance, abrasion resistance and resistance to cutting through which allow it to withstand maltreatment prior to and during use, for example during the course of assembly of a hygiene product.

The combination of wettability and physical properties which are present in the top sheet have been found to result from a process of manufacture in which the grafting reaction is initiated using ultraviolet radiation under conditions which are described in greater detail below. A further advantage of the use of ultraviolet radiation to initiate the graft reaction is that the reaction can be completed surprisingly quickly, for example by exposing the impregnated sheet to radiation for as little as 1 5 seconds, even as little as 5 or 10 seconds or less, and it has been found that the sheet after reaction contains a significant amount of grafted monomer, which can be sufficient for the sheet to be rendered wettable by aqueous solutions. The use of ultraviolet radiation therefore lends itself to continuous processing, which is important in the production of components for hygiene products such as diapers, where efficient and cost effective production is essential for the production process to be viable commercially.

The hydrophilic groups are grafted to sufficient surfaces of the polymeric sheet to enable the sheet to be wetted in its end use application. The groups can be bonded to any combination of the principal external (top and bottom) surfaces of the sheet and the internal pore structure of the sheet. Grafting to surfaces which define the internal pore structure of the sheet can be important for many applications for the sheet to allow satisfactory wicking. For many applications, it will be desirable for the hydrophilic groups to be bonded to one or both of the exposed external surfaces, although it has been found that satisfactory wicking performance is available for many applications when the level of grafting on the exposed external surfaces is less than on the surfaces which define the internal pore structure.

Particular advantages in the top sheet of the hygiene product arise from the fact that the graft reaction between the polymer of the sheet and the hydrophilic monomer takes place while the sheet is impregnated with a solution of the monomer. In particular, it enables great control to be placed over the extent of the grafting reaction, enabling a controllable level of grafting to be obtained on the sheet surfaces (external and within the pores) which can be approximately uniform through the thickness of the sheet, or over the area of the sheet, or both. The presence of the solvent for the monomer can reduce the tendency of monomer to react to produce homopolymer. Furthermore, when such homopolymer is also soluble in the solvent, the presence of the solvent in the reaction system can allow the homopolymer to be washed conveniently from the sheet while in solution, helping to minimise the amount of homopolymer that is deposited on the surfaces of the sheet. It is an important aspect of the present invention that it seeks to provide a graft copolymer between the surface of the sheet and the monomer, while minimising deposition of homopolymer of the monomer on the surfaces (external and internally within the pores) of the sheet. This has the advantage that the hydrophilic properties of the sheet resulting from the graft copolymerisation reaction are less likely to be affected adversely by effects such as exposure of the product to fluids in which homopolymer can dissolve.

The presence of the solvent for the monomer can help to control the temperature to which the sheet is exposed during the reaction, by functioning as a heat sink and getting warmer as the graft reaction proceeds.

Irradiation of the sheet while impregnated with the solution of the monomer has been found to produce a product whose physical properties (for example tensile strength) do not deteriorate on storage, or on exposure to alkaline fluids. Indeed, it has been found that, under some circumstances, the physical properties of the sheet can be enhanced by the treatment, apparently because of the formation of crosslinks in the polymer of the sheet.

The sheet will be formed from a polymeric material which is essentially hydrophobic and which is capable of undergoing the polymerisation reaction with the vinyl monomer on its surface. The hydrophobic nature of the material is such that it is resistant to wetting or is not readily wet, by water (including water based solutions). Such materials can be characterised by a lack of affinity for water. The reaction can increase the affinity of the materials for water (including water based solutions), so as to increase the tendency of the sheet to be wetted by aqueous media. After the reaction, water will tend to spread over the surface of the sheet.

The sheet can include polymers such as for example polyamides and polyesters. Preferred polymeric materials are polyolefins such as polyethylenes and polypropylenes. The sheet can be made from more than just one polymer, either as a mixture of polymers, or with different polymers in different regions of the sheet. It can be made from a polymer which has different physical properties in different regions of the sheet.

The sheet from which the top sheet is made can be a continuous web which has a porous structure to permit transmission of fluid. For example, the sheet can comprise a continuous web in which pores are formed by perforation. The sheet can comprise a web which has a complex three-dimensional porous structure for example as a foam. Porous webs can be made by extraction of one component from a web formed from a mixture of two components. For example, a porous web can be made by extracting polyethylene oxide into warm water (or aqueous solution) from a web that is formed from a mixture of polyethylene and polyethylene oxide.

Preferably, the sheet comprises a fabric formed from fibres whose surface is provided by a hydrophobic polymeric material. The fabric can be woven. Preferably, the fabric is a non-woven fabric. Suitable non-woven fabrics can be made by processes which for example include (a) melt blowing, (b) spinning, (c) wet or dry laying, (d) hydroentangling and (e) needle punching. The fibres of fabrics made by spinning and wet or dry laying can be bonded to one another so that the fabric has integrity, providing mechanical properties which can be required for satisfactory performance. In the case of fabrics made from spun fibres, the fibres can be bonded to one another by the application of heat and pressure, especially in localised regions of the fabric. In the case of fabrics made by wet or dry laying, a lower melting point polymer can be incorporated into the fabric together with the primary polymer, for example as discrete fibres or in bicomponent fibres; bicomponent fibres can be combined with other fibres in a fabric (woven or non-woven), or a fabric can be made completely from bicomponent fibres. For example, in a fabric in which polypropylene is the primary polymer component of the fibres, polyethylene can be introduced for this purpose. A bicomponent fibre, for example in which polymer components are extruded side by side or in a coaxial arrangement, will often have a larger transverse cross-section than a mono-component fibre, which can be a disadvantage for some applications.

Preferably, the mean thickness of the fibres (which might be measured as a mean diameter, especially when the fibres have a circular cross-section) from which a fabric is formed is less than about 30 $\mu$m, more preferably less than about 10 $\mu$m, especially less than about 8 $\mu$m. In the case of certain non-woven fabrics, especially formed by melt blowing or by splitting fibres, the fibre thickness can be thinner for example less than 5 $\mu$m, especially less than 2 $\mu$m. The thickness of the fibres will often be more than about 3 $\mu$m, preferably more than about 5 $\mu$m.

Preferably, the material of the surface of at least some of the fibres of a fabric sheet, for example at least about 40% by weight, preferably at least about 60%, more preferably at least about 80%, comprises a polyolefin, especially polypropylene. Preferably, at least 40% by weight of the material of the fibres of the fabric is polypropylene, more preferably at least about 60%, especially at least about 80%.

Preferably, the material of at least some of the fibres from which a fabric for the sheet is formed, for example at least about 40% by weight, preferably at least about 60%, more preferably at least about 80%, is substantially homogeneous throughout the thickness of the fibres. It can be preferred for many applications for the material of substantially all of the fibres to be substantially homogeneous throughout their thickness, so that those fibres are formed only from polypropylene or another suitable material (with appropriate additives where necessary).

A fabric can be made from fibres comprising more than one material, for example more than one polymer or a polymer having different physical properties in different regions of the fibres or the fabric. For example, the fabric may be made entirely or in part from fibres formed from two polymers such as bicomponent fibres with the components arranged coaxially or side-by-side.

It is particularly preferred that the fabric is formed from fibres which comprise polypropylene alone. This has the advantage that the physical properties of the fabric are those of a non-woven fabric formed from polypropylene fibres which are generally preferred compared with other polyolefin fibres. Compared with bicomponent fibres, the use just of polypropylene fibres has the advantage that the fibres can be made thin without increasing the cost undesirably.

The top sheet in the hygiene product can comprise a laminate formed from a first fabric (as discussed above) and a second non-woven fabric, and optionally additional layers so that the laminate comprises three, four, five or more fabrics. At least one of the fabrics, and preferably each of the fabrics, can have features which are discussed above. In a preferred construction, the first fabric of the laminate can be formed from spun fibres and the second fabric can be a melt-blown fabric. Preferably, the mean thickness of the fibres of the second fabric is not more than about 8 $\mu$m. When the top sheet comprises first and second fabrics, it can be preferred for the ratio of the weight of the fibres of the second fabric to the weight of the fibres of the entire laminate is at least about 0.1.

When the top sheet comprises three or more fabric layers, it can be preferred for the outer layers to be formed from spun fibres (for example spun bonded fibres) and for the, or at least one, layer between them to be a melt-blown fabric. For example, when a top sheet comprises four non-woven fabric layers, the fabrics can be arranged in the order spun bonded:melt-blown:melt-blown:spun bonded.

When the top sheet comprises a laminate, the fabrics can be bonded to one another. They can be bonded by means of an adhesive, or by localised welds.

The effective mean size of pores in the sheet, which are defined by the fibres of the fabric when the sheet is made up of fibres, can be measured using a Coulter porometer. Preferably, the effective mean pore size is less than about 70 $\mu$m, more preferably less than about 50 $\mu$m, especially less than about 40 $\mu$m, for example less than about 30 $\mu$m. In the case of a fabric sheet, such small pore sizes can be attained using small diameter fibres, such as those referred to above.

Preferably, the thickness of the top sheet, measured using test method DIN 53105 which involves lowering a 2.0 kg weight onto a sample of the sheet of area 2.0 cm$^2$ at a speed of 2.0 mm.s$^{-1}$, is greater than about 20 $\mu$m, more preferably greater than about 40 $\mu$m, especially greater than about 60 $\mu$m; preferably, the thickness is less than about 300 $\mu$m, more preferably less than about 200 $\mu$m, especially less than about 125 $\mu$m. The method by which the sheet is made may include a step of calendering the sheet (especially when the sheet is provided by a fabric) to reduce its thickness to a value within the range referred to above, the reduction being by at least about 5%, preferably at least about 15%, more preferably at least about 25%, and less than about 60%, preferably less than about 45%, more preferably less than about 40%. Calendering can have the advantage of reducing the effective size of the pores in the sheet. The calendering step may take place before or after the material of the sheet is reacted with the graft copolymerisation solution. Calendering the sheet before the graft copolymerisation reaction has been found to give rise to increased process speeds.

Preferred monomers are monofunctional acids such as acrylic and methacrylic acids mentioned above, which are able to form thin grafted coatings on the polymeric surface of a sheet. The monomer can exhibit hydrophilic properties when it is grafted to the surface of the hydrophobic polymer. The monomer can be capable of exhibiting hydrophilic properties as a result of a subsequent reaction. For example, the monomer might include a functional group (possibly more than one such group) which can be hydrolysed to form an acid or a salt; for example, it might include be an ester or an amide.

Preferred monomers have a low molecular weight. For example, the molecular weight might be not more than

200 g.mol$^{-1}$, preferably not more than 150 g.mol$^{-1}$, more preferably not more than 100 g.mol$^{-1}$.

The monomer which is used in the graft copolymerisation reaction can be a salt of an acid or is an acid which is capable of being converted to a salt, at least partially, for example by reaction with alkali. It has been found that use of a graft copolymer in salt form (especially the sodium salt or the potassium salt) can give rise to advantages in terms of soft feel (which can be important for user comfort). It can also provide particularly good wicking rates. Furthermore, it is believed that the wettability and wicking rate characteristics that are available in the top sheet of the hygiene product are more stable when the grafted species is in the form of a salt, because the tendency of the surface structure to rearrange by way of "hydrophobic recovery" is reduced.

A further advantage which arises from use of a grafted species is that, at least in some applications, the product can perform ion exchange with ions in solution in the fluid that is to be absorbed, which can reduce the concentration of ions in the fluid. For example, it might be that ammonium ions can be absorbed which can have the advantage of reducing harmful skin reactions.

The extent of the graft reaction to the surface of the hydrophobic polymer sheet can be quantified by measuring the ion exchange capacity of the sheet. The technique for measuring ion exchange capacity is described below. Preferably, the ion exchange capacity measured in milliequivalents per gram (meq.g$^{-1}$) is at least about 0.1, more preferably at least about 0.3, especially at least about 0.6. Preferably, the ion exchange capacity is not more than about 2.0, more preferably not more than about 1.6, especially not more than about 1.4, for example not more than about 1.2. It has been found that useful increases in the physical properties of certain polymer sheets (especially non-woven fabrics which include polypropylene fibres) can be obtained at low graft levels corresponding to these values of the ion exchange capacity.

The solvent for the vinyl monomer will generally be one which does not evaporate to a significant degree in the irradiation step of the method. This has been found to confer the advantage of greater uniformity of properties of the resulting sheet, throughout the thickness of the sheet. Thus there can be greater uniformity in the degree of grafting throughout the thickness of the sheet, leading to efficient wicking of fluid across the area of the sheet. It is believed that this might arise at least in part because of the transparency of the sheet which is retained as a result of the retention of the solvent in the sheet pores. It has also been found that the degree or adverse effects or both of homopolymerisation of the vinyl monomer can be reduced by selection of an appropriate solvent.

Generally, the product of the invention will have a uniform level of grafting through its thickness. This can be achieved by exposure of the sheet to ultraviolet radiation from both sides of the sheet, with sufficient power to penetrate the sheet so that material within the core of the sheet is exposed. However, the product of the invention can be produced so that more of the monomer is grafted on one surface than on the opposite other surface, for example by exposure of the impregnated sheet to ultraviolet radiation from one side only, or predominately from one side. A top sheet which has hydrophilic properties on one surface and hydrophobic properties on the opposite other surface can have advantages when used as a cover sheet in a hygiene product because it can help to maintain dryness against the user's skin.

Preferably, the mean thickness of the grafted vinyl monomer coating is not more than 3.0 μm, more preferably not more than about 2.0 μm, especially not more than about 1.0 μm, for example not more than about 0.5 μm. It is an advantage of the present invention that, by virtue of the graft copolymerisation reaction between hydrophobic polymer surfaces of the sheet and the vinyl monomer, a thin coating can be applied to the surfaces which is uniform (through the thickness of the sheet, or over the area of the sheet, or both) and not susceptible to being removed, for example by dissolution in liquid to which the product is exposed when in use. The ability to provide a coating which is uniform and stable, while also being thin, is important because it means that the reduction in the porosity of the sheet as a result of the graft reaction is minimised. This can be important when the material which is used to treat the sheet swells significantly when exposed to liquid. The uniformity of the coating over the area of the sheet can be identified by assessing variations in the ability of the sheet to be wetted over its area, or by microscopic inspection of the sheet, for example using optical or electron microscopy techniques.

The mean thickness of the grafted vinyl monomer coating can be calculated by determining the amount of vinyl monomer that is present on the surfaces of the hydrophobic polymer sheet in relation to the surface area of the sheet. The amount of the grafted species that is present on the surfaces of the sheet can be determined by measuring the ion exchange capacity of the sheet, when the grafted species can be made to exhibit ion exchange properties. The ion exchange capacity can be measured using standard titration techniques, as described in more detail below. The surface area of the hydrophobic polymer can be determined using optical and electron microscopy techniques. For woven or non-woven substrates this can be done by determining the mean diameter of the different fibres in the substrate and then calculating the total surface area knowing the composition and density of the fibres. Fibre diameters can be measured directly using optical or scanning electron microscopy and standard image analysis. Ten individual diameter readings should be recorded for each type of fibre to obtain an average value. If the fibres are not cylindrical but in the form of ribbons or the like, for example as may occur with splittable fibres, then the mean surface area can be determined from measurements of the cross-section dimensions of the fibre.

The surface area of perforated sheets can also be measured using optical or scanning electron microscope analysis. The mean diameter (average of ten readings) and length of a perforation is measured and the number of perforations per unit weight or area counted. The surface area can then be easily calculated. For foams the surface area can be measured according to the procedure described in US-5387207. The specific surface area of the foam is determined from the equilibrium weight uptake against gravity of a test liquid of known low surface tension, for example absolute ethanol.

The thickness of the layer of grafted material can be calculated from the weight of copolymerized monomer which is grafted on to the substrate. For the case of monomers that have ion exchangeable groups the amount of graft can be calculated from the determination of the ion exchange capacity (see as discussed below). The weight of grafted monomer per gram of grafted substrate is calculated according to the equation:

$$Weight\ (g)\ =\ \frac{IEC \times EW}{1000} \times k$$

where IEC is the ion exchange capacity (meq.$g^{-1}$), EW is the equivalent weight of the monomer (72.06 for acrylic acid), and k is a conversion factor which accounts for the fact that not all of the functional groups of the vinyl monomer which can participate in an ion exchange reaction are exchanged during the determination of the ion exchange capacity.

The value of the conversion factor ("k") can be calculated by determining the graft level for a representative sample using both the ion exchange capacity method and the gravimetric method (as discussed in more detail below). The value is then calculated using the equation:

$$k\ =\ \frac{W_g}{IEC \times EW} \times 1000$$

where $W_g$ is the weight of grafted monomer per gram of grafted substrate, determined gravimetrically.

For monomers that do not have ion exchange groups then the amount of graft can be determined gravimetrically. A sample of substrate (top sheet) is weighed and then graft copolymerized according to the methods described in this specification. The sample is then re-weighed. The weight of grafted monomer per gram of grafted substrate is calculated according to the equation:

$$Weight\ (g)\ =\ \frac{W_1 - W_0}{W_1}$$

where $W_1$ is the weight in grams of the grafted substrate and $W_0$ is the weight in grams of the ungrafted substrate.

The mean coating thickness of the graft copolymerized monomer can then be determined using the following equation:

$$Thickness\ (\mu m)\ =\ \frac{10000 \times W_g}{\rho_m \times A_s (1 - W_g)}$$

where $W_g$ is the weight of grafted polymer per gram of grafted substrate, $\rho_m$ is the density of the polymerized monomer (0.94 g.$cm^{-3}$ for polyacrylic acid) and $A_s$ is the surface area of one gram of non-grafted substrate.

In another aspect, the invention provides an absorbent hygiene product which comprises a core of an absorbent material and a top sheet through which fluid to be absorbed passes for absorption by the absorbent material, in which the top sheet is porous and comprises a sheet having surfaces provided by a hydrophobic polymeric material which have been modified by a graft copolymerisation reaction between the surfaces and a vinyl monomer which is an ethylenically unsaturated acid or an ester thereof so that the monomer exhibits or can be changed so that it exhibits hydrophilic properties, and in which the grafted vinyl monomer is provided as a substantially uniform coating on the sheet surfaces whose mean thickness not more than 3.0 $\mu$m.

The vinyl monomer which is graft copolymerised with the surface of the polymer sheet can be capable of reacting with an acid or a base directly to form a salt, or indirectly to form a salt after appropriate work up, perhaps involving for example hydrolysis or sulphonation. Preferred vinyl monomers include ethylenically unsaturated carboxylic acids and esters thereof such as acrylic acid (and salts thereof), methacrylic acid (and salts thereof), methyl acrylate, and methylmethacrylate.

Preferred monomers are monofunctional acids such as acrylic and methacrylic acids mentioned above, which are able to form thin grafted coatings on the polymeric surface of a sheet. The monomer can exhibit hydrophilic properties when it is grafted to the surface of the hydrophobic polymer. The monomer can be capable of exhibiting hydrophilic properties as a result of a subsequent reaction. For example, the monomer might include a functional group (possibly more than one such group) which can be hydrolysed to form an acid or a salt; for example, it might include an ester or an amide.

Suitable solvents for use in making the top sheet will generally be transparent to ultraviolet radiation, have no atoms which are abstractable when exposed to radiation, have a high specific heat and a high latent heat of vaporisation, and will not react adversely with the material of the sheet. A suitable solvent can comprise a mixture of solvent components, including one component in which the vinyl monomer dissolves more readily. A preferred solvent (or solvent component of a solvent mixture) will have a boiling point which is greater than about 50°C, preferably greater than about 70°C. It is also preferred that the boiling point of the solvent (or solvent component of a solvent mixture) is no higher than a temperature at which the fabric might be damaged during ultraviolet irradiation. For example, the boiling point of the solvent (or solvent component of a solvent mixture) might be selected to be less than the temperature at which the polymer material of the sheet melts or softens. Particularly preferred solvents have a latent heat of vaporisation which is greater than about 1000 $J.g^{-1}$, preferably greater than about 1500 $J.g^{-1}$, more preferably greater than about 2000 $J.g^{-1}$, and/or a specific heat capacity which is greater than about 2.0 $J.g^{-1}.K^{-1}$, preferably greater than about 3.0 $J.g^{-1}.K^{-1}$, more preferably greater than about 4.0 $J.g^{-1}.K^{-1}$. A value of specific heat capacity, or of latent heat of vaporisation, within these ranges has the advantage that the solvent in the reaction has an enhanced ability to dissipate heat without evaporating to a significant degree, giving rise to the advantages referred to above. A particularly significant further advantage is that the formation of product from the homopolymerisation reaction of the vinyl monomer is restricted, and any such product which is formed is retained in solution rather than being deposited in pores within the sheet. This allows the product to be removed easily from the sheet by washing. The control over the formation of the homopolymerisation product can be achieved without use of inhibiting agents, which can cause contamination problems when the sheet is in use in certain applications. It is particularly preferred that the solvent comprises water.

Preferably, solvent in the vinyl monomer solution comprises at least about 50% water, more preferably at least about 60%, especially at least about 70%, particularly at least about 85%, based on the total weight of the solution apart from the vinyl monomer component or components, and any other significant functional components. The solvent can comprise one or more organic solvents, for example selected from the group consisting of alcohols, ketones and ethers. Preferably, the organic solvent is miscible in water. A suitable solvent might be a low molecular alcohol, for example ethanol or propan-2-ol. When the solvent system includes an organic solvent, the amount of the organic solvent in the solvent system, based on the total weight of the solution (apart from the vinyl monomer component or components and any other significant functional components) will preferably be not more than about 50%, more preferably not more than about 40%, especially not more than about 30%. Preferably, the amount of the organic solvent is at least about 5%, more preferably at least about 10%, for example at least about 15%.

The impregnation solution will often include an initiator for the graft reaction. Preferably, the initiator initiates the reaction by abstracting an atomic species from one of the reacting materials, especially by abstracting an atom from the polymer of the sheet surface to form a polymer radical on the sheet surface. For example it can be particularly preferred to use an initiator which can abstract a hydrogen atom from the polymer of the sheet surface. When an atom is abstracted from the polymer of the sheet, the activated polymer species can react either with another polymer group in the sheet so that the polymer becomes crosslinked, or with the vinyl monomer in a copolymerisation reaction. The copolymerisation reaction results in the formation of a grafted branch.

Examples of suitable initiators include benzophenone and substituted benzophenones such as 2, 3 or 4-methylbenzophenone, methyl-2-benzoylbenzoate, 4-chlorobenzophenone, 4-phenylbenzophenone, 4-benzoyl-4'-methyldiphenyl sulphide and mixtures thereof. Other suitable initiators include thiozanthone and substituted thiozanthones, for example 2,4-dimethylthiozanthone, 2-chlorothiozanthone, 2-isopropylthiozanthone 4-isopropylthiozanthone, 1-chloro-4-propoxythiozanthone and mixtures thereof.

The mole ratio of the vinyl monomer to the initiator is preferably at least about 40, more preferably at least about 75, especially at least about 125; the ratio is preferably less than about 1500, more preferably less than about 1000, especially less than about 350, more especially less than about 250; for example the ratio may be about 150.

The impregnation solution may include a component by which homopolymerisation of the vinyl monomer is inhibited. Examples of suitable inhibitors include iron (II) and copper (II) salts which are soluble in the reaction medium, a preferred material for aqueous media being copper (II) sulphate. It has been found, however, that the need for an inhibitor can be avoided by selection of an appropriate solvent for the graft copolymerisation reaction which can restrict the speed and degree of the homopolymerisation reaction, for example as a result of its ability to act as a heat sink. This can be an advantage when it is desired to minimise the amount of contaminants in the sheet.

The impregnation solution can include additional components to optimise reaction conditions such as surfactants to ensure that the solution fully impregnates the sheet, an appropriate mixture of solvents to ensure homogeneity of the solution, and so on.

The sheet can be treated mechanically to encourage impregnation of the sheet by the solution, for example ultrasonically.

It will often be appropriate to restrict exposure of the impregnated sheet to oxygen, at least during exposure of the sheet to ultraviolet radiation. This can be achieved for example by carrying out the ultraviolet irradiation step in an inert atmosphere, for example in an atmosphere of argon or nitrogen, or sealing the impregnated fabric between sheets of material which are impervious to oxygen, but are transparent to ultraviolet radiation of appropriate wavelength for initiating the co-polymerisation reaction.

The hygiene product of the invention finds particular application as a diaper. Other applications include for example sanitary napkins, tampons, incontinence pads, and training pants. It can be used for medical applications such as a bandage and a wound dressing. It can be used for cleaning applications for example as wipes. The product will generally be disposable in that it is not generally intended to be laundered or otherwise restored for reuse. Suitable absorbent material for the core of the hygiene product of the invention will depend on its intended application. Examples of suitable materials include those disclosed in US-4950264, US-4610678, US-4834735, US-4673402 and US-4888231.

In another aspect, the invention provides a method of making an absorbent hygiene product comprising a core of an absorbent material and a top sheet through which fluid to be absorbed passes for absorption by the absorbent material, which comprises:

a. impregnating a porous sheet, having surfaces which are provided by a hydrophobic polymeric material, with a solution of a vinyl monomer which is an ethylenically unsaturated acid or an ester thereof so that the monomer exhibits or can be changed so that it exhibits hydrophilic properties,
b. exposing the impregnated sheet to ultraviolet radiation to cause the vinyl monomer to copolymerise with the material of the sheet,
c. drying and finishing the sheet to product a top sheet, and
d. laminating the top sheet with a core of adsorbent material.

The technique used to assemble the product will depend on the materials from which it is made, the number of component layers, the application for the product and so on. The hygiene product might comprise two or more top sheets of which at least one has features which are discussed in detail above. For example, a hygiene product might include two top sheets of which one is intended to distribute fluids across the surface of the core of absorbent material, and the other is intended to pass fluid quickly from one side in contact with the user's skin towards the core of absorbent material on the other side. One or both of these top sheets might have the features that are discussed above resulting from the ultraviolet radiation initiated graft copolymerisation reaction.

Generally, the hygiene product will also include a back sheet. The back sheet will usually be generally impervious to fluid that has been adsorbed by the core.

The hygiene product can include fasteners by which it can be held in place against a user's skin. The nature of the fasteners will depend on the nature of the product and its intended application. A diaper can include adhesive tape fasteners. A sanitary napkin can include an adhesive strip. Products such as diapers, sanitary napkins and incontinence pads can include elasticated borders to facilitate accurate location and to minimise leakage.

Suitable techniques for constructing hygiene products with top sheets are well known. The lamination of the top sheet and the core might involve fixing the top sheet to the core, possibly over the entire area of the two components, or over just a part of their areas (for example around their edges), for example by means of an additional adhesive component (for example a hot-melt adhesive), or by localised application of heat and pressure (for example to form a plurality of pinbonds). The lamination of the top sheet and the core might involve just aligning the two and then relying on other components of the hygiene product to hold them relative to one another, such as fastening tapes, and a back sheet which is wrapped around the edges of the top sheet and the core.

The hygiene product can include other components according to the application for which it is to be used. In particular, it can include a back sheet on the face which is opposite to the top sheet. The back sheet will generally prevent transmission of fluid so that fluid absorbed by the material of the core is retained within the product. The product might also include for example means for fastening it in place (such as adhesive tabs in the case of a diaper).

In the examples that follow, data are provided relating to the ion exchange capacity weight loss, surface tension of extract solution, aging and solvent strike through. These parameters were measured using the following techniques.

### Ion exchange capacity

The extent of the graft reaction to the surface of the hydrophobic polymer sheet can be quantified by measuring the ammonium ion exchange capacity of the sheet. It is measured in milliequivalents per gram according to a test routine in which a sample of the sheet weighing about 0.5 g is converted into the acid (H⁺) form by immersion in 1.0 M hydrochloric acid at 70°C for 1 hour. The sample is washed in deionised water until the washing water shows a pH in the range of about 6 to 7. The sample is then dried to constant weight at 70°C.

The dried sample is placed in a 100 ml polyethylene bottle to which is added accurately 10 ml of approximately 0.1 M ammonium hydroxide. Additional deionised water can be added to immerse the sample fully. A further 10 ml of ammonium hydroxide is added to a second polyethylene bottle, together with the same amount of deionised water as that added to the bottle containing the sample. Both bottles are stored at 70°C for at least 30 minutes.

After being allowed to cool, the contents of each bottle are transferred to glass conical flasks, and the amount of ammonium hydroxide in each is determined by titration with standardised 0.1 M hydrochloric acid, using a methyl red indicator.

The ion exchange capacity (in meq.g$^{-1}$) of the treated sheet in the dry acid (H⁺) form is calculated according to the equation:

$$IEC = \frac{t_2 - t_1}{10W}$$

where $t_1$ is the titration value in ml of HCl from the bottle with the sample, $t_2$ is the titration value in ml of HCl from the bottle without the sample, and W is the weight in grams of the dried membrane in acid (H⁺) form.

### Vertical wicking height

Strips of treated sheet, 15 mm x 150 mm, are cut from the centre of the web with the longest dimension being parallel to the machine direction. The strip is suspended over a dish containing a saline solution (0.9% sodium chloride in de-ionized water) and then lowered so that 5 mm of the sample is below the surface of the solution. A timer was started and the height the moving liquid front reached after 10 minutes was recorded.

### Weight loss

A sample of treated sheet (for example approximate weight 0.5 g) is dried to constant weight at 70°C and then placed into a conical flask containing 1.5 litres of boiling deionized water. With continuous stirring the sample is exposed to the boiling water for 4 hours. The sample is then removed and dried to constant weight at 70°C and re-weighed. Samples in their potassium or sodium form are soaked for a further one hour in 1M potassium or sodium hydroxide at 70°C before being rinsed in de-ionised water and dried to constant weight at 70°C. The percentage weight loss is calculated thus:

$$Weight\ loss\ (\%) = \frac{W_1 - W_2}{W_1} \times 100$$

where:

$W_1$ is the initial weight of sample (g).
$W_2$ is the weight of sample (g) after immersion in boiling water.

### Surface tension measurement of extract solution

A sample of treated sheet 100 mm $\times$ 100 mm is extracted for 4 hours in 50ml of de-ionized water at 70°C. The fabric is then removed and the extract water allowed to cool to room temperature (20 to 25°C). The surface tension of the extract water is measured with a Du Nouy surface tension balance (Model C440890 - Cambridge Instruments) using the ring method and compared with the value for pure water, 72 mN.m$^{-1}$ at 23°C (Handbook of Chemistry and Physics,

59th Edition, published CRC Press).

Aging

Samples of treated sheet 15 mm × 150 mm are stored at 70°C in an oven. Every 24 hours a sample is removed and the vertical wicking height measured using the procedure described previously.

Strike through test

The EDANA recommended test method 150.3-96 is applied as appropriate. The following adaptations were incorporated in the examples below:

- the reference pads used were 10cm × 10cm squares of Air-Laid Wiper 7002, 1 ply white paper (supplied by Cotswold Safety and Hygiene, UK). Seven layers are used with the smooth surface facing up.
- laboratory temperature was controlled to 23°C.
- laboratory humidity varied between 48% and 65%.

The following examples relate to top sheets which are suitable for use in hygiene products of the kind with which the present invention is concerned. They can be combined with absorbent material cores using conventional techniques and materials.

DETERMINATION OF CONVERSION FACTOR (k)

A non-woven polypropylene fabric, supplied by Fibertex A/S of Denmark, made from a thermally bonded laminated construction of spun bonded:meltblown:meltblown:spun bonded (SMMS) was selected. The spunbonded component had a mean fibre diameter of 15 to 20 $\mu$m and a basis weight of 4 $g.m^{-2}$, and the meltblown component had a mean diameter of 6 $\mu$m and a basis weight of 1 $g.m^{-2}$. The total basis weight of the fabric was 10 $g.m^{-2}$ and the nominal thickness was 90 $\mu$m. The surface area of the fibres of the fabric was 3525 $cm^2.g^{-1}$.

A sample strip of the fabric, 152 mm wide and about 14 m long, was accurately weight. The sample was joined at each end with further lengths of a standard 50 $g.m^{-2}$ polypropylene non-woven spun bonded fabric. The combined length of the fabric strip was about 40 m.

The strip of fabric was impregnated in a continuous process with a solution formulated as follows (percentage by weight) by passing the fabric around rollers located in a chamber with an atmosphere of nitrogen, so that the fabric passes through a solution consisting of:

| | |
|---|---|
| Acrylic acid (99%, Sigma-Aldrich Company Limited, UK) | 30.00% |
| Benzophenone (99%, Lambson Fine Chemicals Limited, UK) | 0.25% |
| 2-propanol (99+%, Sigma-Aldrich Company Limited, UK) | 10.00% |
| De-ionized water | 59.75% |

The mole ratio of acrylic acid to benzophenone was about 150.

The impregnated fabric, still in a nitrogen atmosphere, was passed between four medium pressure mercury lamps, positioned parallel to one another, two each side of the chamber, the chamber at that point being provided by quartz windows. Each of the lamps had a power output of 120 $W.cm^{-1}$ and was positioned 10 cm from the fabric. Each lamp produced a parallel beam having a width of 10 cm. The total exposure time of the fabric to the radiation was about 12 seconds.

Still as part of the continuous process, the graft copolymerized fabric was washed to remove unreacted components by passing through two tanks of de-ionized water. The total wash time in the de-ionized water was about 10 minutes. The fabric was finally dried over heated rollers at 60°C.

A further sample of fabric was graft copolymerized using the procedure described above using a total exposure time to the UV radiation of about 6.3 seconds, and the total wash time in the de-ionized water was about 10 minutes.

The treated samples were carefully detached from the standard spun bonded material and accurately re-weighed. The ion exchange capacity of the two samples was measured and the conversion factors (k) calculated according to the procedure described above. The results of these calculations are shown below:

| UV irradiation time (s) | 12 | 6.3 |
|---|---|---|
| Weight of untreated fabric $W_0$ (g) | 20.0703 | 22.1253 |
| Weight of graft copolymerised fabric $W_1$ (g) | 25.4228 | 25.1540 |
| Weight of grafted monomer per gram of grafted substrate $W_g$ | 0.211 | 0.0444 |
| Ion exchange capacity (meq.g$^{-1}$) | 2.54 | 0.54 |
| Conversion factor.k | 1.15 | 1.14 |

As can be seen the conversion factor (k) for both examples is effectively the same and shows that the same value can be used over a large range of ion exchange capacity measurements to determine the weight of grafted monomer per gram of grafted substrate. In the examples below the value of the conversion factor will be taken as 1.145.

EXAMPLE 1

A continuous strip of the non-woven fabric, which was prepared using the method described above in relation to the determination of the conversion factor, was impregnated with a solution formulated as follows (percentage by weight) by passing the fabric around rollers located in a chamber with an atmosphere of nitrogen, so that the fabric passed through a solution consisting of (proportions in weight percent):

| | |
|---|---|
| Acrylic acid | 30-00% |
| Benzophenone | 0.25% |
| Lutensol ON-70 surfactant | 0.50% |
| De-ionized water | 69.25% |

The mole ratio of acrylic acid to benzophenone was about 150.

The impregnated fabric, still in a nitrogen atmosphere, was passed between four medium pressure mercury lamps, positioned parallel to one another, two each side of the chamber, the chamber at that point being provided by quartz windows. Each of the lamps had a power output of 79 W.cm$^{-1}$ and was positioned 10 cm from the fabric. Each lamp produced a parallel beam having a width of 10 cm. The total exposure time of the fabric to the radiation was about 8.5 seconds.

Still as part of the continuous process, the graft copolymerised fabric was washed to remove unreacted components by passing it through two tanks of deionised water. The total wash time in the deionised water was about 7 minutes.

The properties of the graft copolymerized fabric are set out below, and compared with the corresponding properties of the polypropylene fabric starting material.

| Property | Ungrafted | Grafted |
|---|---|---|
| Ion exchange capacity (meq.g$^{-1}$) | 0 | 0.58 |
| Weight of grafted monomer per gram of grafted substrate | 0 | 0.048 |
| Vertical wicking height (mm) | 0 | 23 |
| Weight loss (%) | 0.38 | 1.04 |
| Strike through (s) | 29.77 | 3.07 |
| Surface tension of extract water (mN.m$^{-1}$) | 72 | 63.5 |
| Mean graft coating thickness ($\mu$m) | 0.0 | 0.15 |

EXAMPLE 2

The non-woven fabric described above was graft polymerized with acrylic acid according to the procedure described in Example 1 with the following differences. The lamp power was 120 W.cm$^{-1}$, the irradiation time was 7 seconds and the impregnation solution was the same as that used in the method described above for determining the conversion factor

The properties of the graft polymerized fabric are set out below, and compared with the corresponding properties with of the polypropylene fabric starting material.

| Property | Ungrafted | Grafted |
|---|---|---|
| Ion exchange capacity (meq.g$^{-1}$) | 0 | 0.96 |
| Weight of grafted monomer per gram of grafted substrate | 0 | 0.079 |
| Vertical wicking height (mm) | 0 | 31 |
| Weight loss (%) | 0.38 | 0.23 |
| Strike through (s) | 29.77 | 2.86 |
| Surface tension of extract water (mN.m$^{-1}$) | 72 | 69.1 |
| Mean graft coating thickness ($\mu$m) | 0 | 0.26 |

As can be seen the graft copolymerized fabric produced in this example shows much lower weight loss and the extract water shows a significantly lower surface tension reduction than for Example 1.

EXAMPLE 3

A strip of fabric made according to Example 2 was converted into the potassium form by passing the fabric through a 10% w/w potassium hydroxide solution at room temperature. The total immersion time was about 6 seconds. The fabric was then washed to remove unreacted components and dried on two rollers at 60°C.
The properties of the treated fabric are set out below, and compared with the corresponding properties of the fabric of Example 2.

| Property | Example 2 | Example 3 |
|---|---|---|
| Vertical wicking height (mm) | 31 | 68 |
| Weight loss (%) | 0.23 | 0.95 |
| Strike through (s) | 2.86 | 2.98 |
| Surface tension of extract water (mN.m$^{-1}$) | 69.1 | 67.9 |

EXAMPLE 4

The procedure of Example 3 was repeated except the solution was 30% w/w sodium hydroxide and the immersion time was 10 minutes. The properties of the treated fabric, now substantially in the sodium form, are set out below.

| Property | |
|---|---|
| Vertical wicking height (mm) | 46 |
| Weight loss (%) | 0.5 |
| Strike through (s) | 2.99 |
| Surface tension of extract water (mN.m$^{-1}$) | 72 |

As can be seen from Examples 3 and 4, converting into the potassium or sodium form significantly improves vertical wicking height without adversely effecting the other properties.

EXAMPLE 5

Samples of fabric made according to Examples 2, 3 and 4 were subject to a number of washing cycles as described previously in the Weight Loss procedure. After each wash the vertical wicking height after 10 minutes and strike through were measured. The results are given in the following table:

| Number of washes | Example 2 | | Example 3 | | Example 4 | |
|---|---|---|---|---|---|---|
| | Vertical wicking height (mm) | Strike through (s) | Vertical wicking height (mm) | Strike through (s) | Vertical wicking height (mm) | Strike through (s) |
| 0 | 31 | 2.86 | 68 | 2.98 | 46 | 3.66 |
| 1 | 49 | 2.99 | 50 | 3.21 | 45 | 2.89 |
| 2 | 55 | 2.95 | 41 | 2.88 | 40 | 2.3 |
| 3 | 35 | 2.35 | 35 | 3.04 | 30 | 2.86 |

As can be seen the fabric treated according to the present invention shows excellent durability to repeated washings.

EXAMPLE 6

Samples of treated fabric made according to Examples 3 and 4 were subjected to the Aging test described above. The results are shown in the table below.

| Aging (days) | Vertical wicking height (mm) | |
|---|---|---|
| | Example 3 | Example 4 |
| 0 | 68 | 46 |
| 2 | 60 | 43 |
| 7 | 55 | 31 |

As can be seen the treated fabrics have good aging properties.

EXAMPLE 7

The procedure described in Example 2 above was repeated at a number of different irradiation times so as to produce a series of graft copolymerizes fabrics with a range of mean coating thickness.
The properties of the graft copolymerized fabrics are set out below:

| Property | UV irradiation time (seconds) | | | | | | |
|---|---|---|---|---|---|---|---|
| | 5.5 | 6.4 | 7.1 | 8.0 | 9.2 | 10.9 | 13.3 |
| Ion Exchange Capacity (meq.g$^{-1}$) | 0.44 | 0.72 | 0.91 | 1.27 | 2.28 | 3.13 | 4.02 |
| Weight of grafted monomer per gram of grafted substrate (g.g$^{-1}$) | 0.036 | 0.059 | 0.075 | 0.105 | 0.188 | 0.258 | 0.332 |
| Graft coating thickness ($\mu$m) | 0.11 | 0.19 | 0.25 | 0.35 | 0.70 | 1.05 | 1.50 |
| Vertical wicking height (mm) | 0 | 16 | 36 | 43 | 10 | 16 | 15 |
| Strike through (s) | 3.35 | 3.29 | 3.27 | 3.21 | 3.72 | 3.92 | 4.27 |

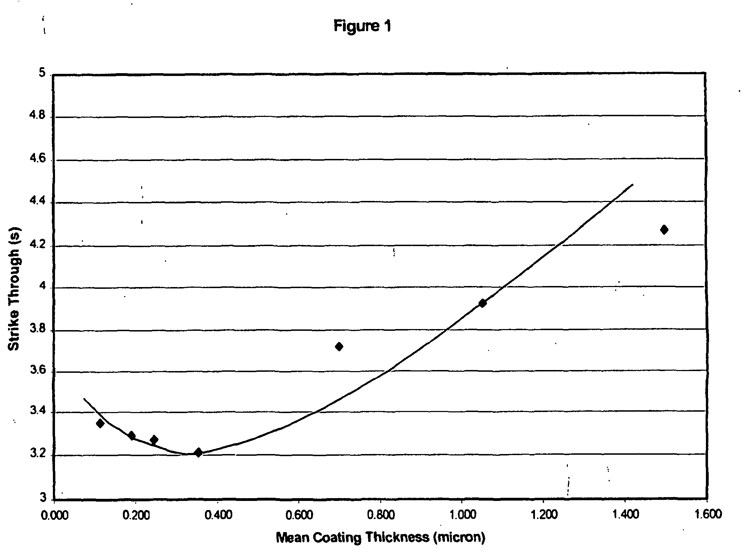

The data shows that the quickest strike through and greatest vertical wicking height is achieved at a mean coating thickness of about 0.35 $\mu$m. As the coating thickness increases above this value strike through and vertical wicking height performance begins to decrease. This effect on the strike through times is shown in Figure 1.

EXAMPLE 8

A non-woven polypropylene spun fibre fabric supplied by Nippon Kodoshi Corporation, Japan was selected. The fabric had a basis weight of 46 $g.m^{-2}$ and a nominal thickness of 100 $\mu$m The mean fibre diameter was 11.3 $\mu$m. The surface area of the fibres of the fabric was 3900 $cm^2.g^{-1}$.

A sample of the fabric, 10cm x 10 cm square, was accurately weight and then impregnated with a solution formulated as follows (percentage by weight) by immersing the fabric in the solution until completely wetted out. The solution was formulated as follows:

| | |
|---|---|
| N, N-dimethylacrylamide (99% Sigma-Aldrich Company Ltd, UK) | 30.00% |
| Benzophenone | 0.25% |
| Lutensol ON-70 surfactant | 0.50% |
| De-ionized Water | 69.25% |

The mole ratio of N, N-dimethylacrylamide to benzophenone was about 220.

The impregnated fabric was placed between two pieces of polyethylene film (thickness 50 $\mu$m) and all the air excluded. The fabric and polyethylene film was then placed between two pieces of quartz glass. Each side of the fabric sheet was then sequentially exposed for 30 seconds per side to UV radiation from a medium pressure mercury lamp positioned 22 cm from the sample. The mercury lamp had a power output of 43 $W.cm^{-1}$.

The treated fabric was then washed in boiling de-ionized water for 20 minutes, dried at 70°C and re-weighed.

Further samples of treated fabric were prepared according to the procedure above using UV exposure times of 60, 90, 120 and 150 seconds per side. -

The properties of the graft copolymerized fabrics are set out below. The coating thickness was calculated from the gravimetrically determined graft weight.

| Property | UV irradiation time (seconds per side) | | | | |
|---|---|---|---|---|---|
| | 30 | 60 | 90 | 120 | 160 |
| Weight of grafted monomer per gram of grafted substrate ($g.g^{-1}$) | 0.0047 | 0.0095 | 0.0420 | 0.1469 | 0.1966 |
| Graft coating thickness ($\mu$m) | 0.01 | 0.03 | 0.12 | 0.46 | 0.65 |
| Weight loss (%) | 0.33 | 0.41 | 0.14 | 0.48 | 0.73 |
| Vertical wicking height (mm) | 10 | 60 | 45 | 40 | 2 |

As can be seen the graft copolymerized fabric produced using this monomer shows low weight loss and good vertical wicking height up to a mean coating thickness of about 0.46 $\mu$m. Above a mean coating thickness of about 0.6 $\mu$m the vertical wicking height decreases.

EXAMPLE 9

A non-woven polypropylene meltblown fabric supplied by Applied Extrusion Technologies Inc USA was selected. The fabric had a basis weight of 13 $g.m^{-2}$ and a nominal thickness of 40 $\mu$m. The mean fibre diameter was 3.5 $\mu$m. The surface area of the fibres of the fabric was 12,560 $cm^2.g^{-1}$.

A continuous strip of the fabric was graft copolymerised with acrylic acid according to the procedure described in Example 1 with the following differences. The lamp power was 59 $W.cm^{-1}$, the irradiation time was 6 seconds, and the wash time in deionized water was 5 minute

The treated fabric had an ion exchange capacity of 1.19 $meq.g^{-1}$, the weight of grafted monomer per gram of grafted substrate was 0.098 $g.g^{-1}$ and the mean coating thickness was 0.09 $\mu$m.

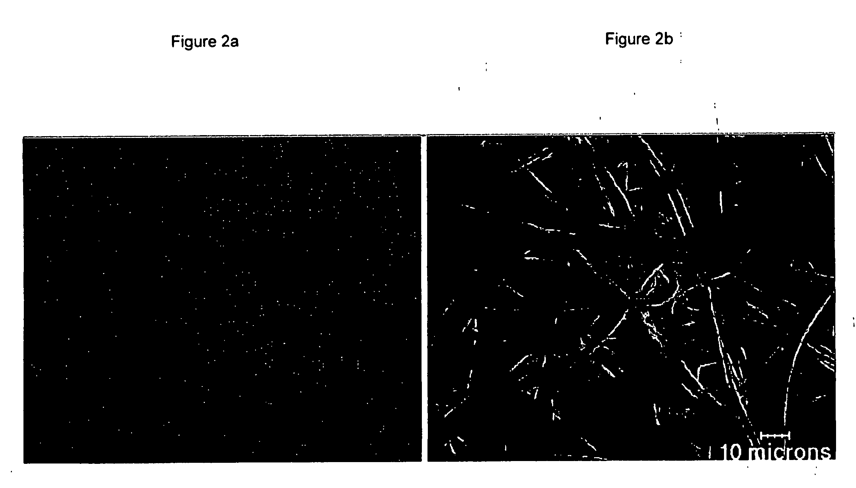

The treated fabric was converted into the potassium form according to the procedure described in Example 3. Using a scanning electron microscope the distribution of the acrylic acid on the surface of the fibres was determined by potassium ion mapping using Energy Dispersive X-ray analysis (EDX analysis). Figure 2a shows the results of this analysis for an area of the fabric. Figure 2b is a SEM photograph of the same area of that fabric. As can be seen the potassium ions (shown as white dots) and therefore the grafted acrylic acid are evenly distributed over the fibre surface.

**Claims**

1. An absorbent hygiene product which comprises a core of an absorbent material and a top sheet through which fluid to be absorbed passes for absorption by the absorbent material, in which the top sheet is porous and comprises a sheet having surfaces provided by a hydrophobic polymeric material which have been modified by a graft copolymerisation reaction between the surfaces and a vinyl monomer which is an ethylenically unsaturated acid or an ester thereof so that the monomer exhibits or can be changed so that it exhibits hydrophilic properties, and in which the product is made by a process which involves exposure of the sheet to ultraviolet radiation while impregnated with a solution of the vinyl monomer.

2. A hygiene product as claimed in claim 1, which contains trace quantities of an initiator for the graft copolymerisation reaction by which an atomic species has been abstracted from the polymer of the sheet surface to form a polymer radical on the sheet surface.

3. A hygiene product as claimed in claim 1, in which groups that are bonded to the surface of the hydrophobic polymeric material comprise a salt of an ethylenically unsaturated acid.

4. A hygiene product as claimed in claim 1, in which the polymeric material of the sheet comprises at least one polyolefin.

5. A hygiene product as claimed in claim 1, in which the polymeric material of the sheet comprises polypropylene.

6. A hygiene product as claimed in claim 1, in which the mean thickness of the top sheet is less than 400 $\mu$m.

7. A hygiene product as claimed in claim 1, in which the sheet comprises a fabric formed from fibres whose surface is provided by a hydrophobic polymeric material.

8. A hygiene product as claimed in claim 7, in which at least 40% by weight of the material of the fibres of the fabric is polypropylene.

9. A hygiene product as claimed in claim 7, in which the mean thickness of the fibres from which the fabric of the top sheet is formed is less than 30 $\mu$m.

10. A hygiene product as claimed in claim 7, in which the top sheet comprises a laminate formed from first and second non-woven fabrics.

11. A hygiene product as claimed in claim 10, in which the first fabric of the laminate is formed from spun fibres and the second fabric is a melt-blown fabric.

12. A hygiene product as claimed in claim 10, in which the mean thickness of the fibres of the second fabric is not more than 8 $\mu$m.

13. A hygiene product as claimed in claim 10, in which the ratio of the weight of the fibres of the second fabric to the weight of the fibres of the entire laminate is at least 0.1.

14. A hygiene product as claimed in claim 10, in which the laminate includes a third fabric.

15. A method of making an absorbent hygiene product comprising a core of an absorbent material and a top sheer through which fluid to be absorbed passes for absorption by the absorbent material, which comprises:

   a. impregnating a porous sheet, having surfaces which are provided by a hydrophobic polymeric material, with a solution of a vinyl monomer which is an ethylenically unsaturated acid or an ester thereof so that the monomer exhibits or can be changed so that it exhibits hydrophilic properties,
   b. exposing the impregnated sheet to ultraviolet radiation to cause the vinyl monomer to copolymerise with the material of the sheet,
   c. drying and finishing the sheet to produce a top sheet, and
   d. laminating the top sheet with a core of adsorbent material.

16. A method as claimed in claim 15, in which the solvent for the vinyl monomer does not evaporate significantly during the step of exposing the impregnated sheet to ultraviolet radiation.

17. A method as claimed in claim 15, in which the solvent for the vinyl monomer comprises at least 50 wt-% water, based on the total weight of the solution apart from the vinyl monomer component or components, and any other significant functional components.

18. A method as claimed in claim 15, in which the solution for the vinyl monomer comprises at least 5 wt.% of an organic solvent.

19. A method as claimed in claim 15, which includes the step of treating the sheet mechanically to encourage impregnation of the sheet by the vinyl monomer solution.

20. A method as claimed in claim 15, in which the vinyl monomer solution contains an initiator which abstracts an atomic species from the polymer of the sheet surface to form a polymer radical on the sheet surface.

21. A method as claimed in claim 15, in which the vinyl monomer is an acid, and the method includes the step of neutralising the acid, at least partially to convert the acid to a salt, preferably a sodium salt or a potassium salt.

22. An absorbent hygiene product which comprises a core of an absorbent material and a top sheet through which fluid to be absorbed passes for absorption by the absorbent material, in which the top sheet is porous and comprises a sheet having surfaces provided by a hydrophobic polymeric material which have been modified by a graft copolymerisation reaction between the surfaces and a vinyl monomer which is an ethylenically unsaturated acid or an ester thereof so that the monomer exhibits or can be changed so that it exhibits hydrophilic properties, and in which the grafted vinyl monomer is provided as a substantially uniform coating on the sheet surfaces whose mean thickness is not more than 3.0 $\mu$m.

**Patentansprüche**

1. Absorbierendes Hygieneprodukt, das einen Kern aus einem absorbierenden Material und eine obere Folie umfasst, durch welche zu absorbierendes Fluid zur Absorption durch das absorbierende Material tritt, worin die obere Folie porös ist und eine Folie mit Oberflächen umfasst, die durch ein hydrophobes polymeres Material bereitgestellt werden und durch eine Pfropf-Copolymerisationsreaktion zwischen den Oberflächen und einem Vinyl-Monomer modifiziert worden sind, bei dem es sich um eine ethylenisch ungesättigte Säure oder einen Ester derselben handelt, so dass das Monomer hydrophile Eigenschaften zeigt oder so abgeändert werden kann, dass es diese zeigt, und wobei das Produkt durch ein Verfahren hergestellt ist, das die Einwirkung von Ultraviolettstrahlung auf die Folie beinhaltet, während sie mit einer Lösung des Vinyl-Monomers imprägniert wird.

2. Hygieneprodukt nach Anspruch 1, das Spurenmengen eines Initiators für die Pfropf-Copolymerisationsreaktion enthält, durch welchen eine atomare Spezies von dem Polymer der Folienoberfläche abstrahiert worden ist, um ein Polymer-Radikal auf der Folienoberfläche zu bilden.

3. Hygieneprodukt nach Anspruch 1, in dem Gruppen, die an die Oberfläche des hydrophoben polymeren Materials gebunden sind, ein Salz einer ethylenisch ungesättigten Säure umfassen.

4. Hygieneprodukt nach Anspruch 1, in dem das polymere Material der Folie mindestens ein Polyolefin umfasst.

5. Hygieneprodukt nach Anspruch 1, in dem das polymere Material der Folie Polypropylen umfasst.

6. Hygieneprodukt nach Anspruch 1, in dem die mittlere Dicke der oberen Folie weniger als 400 $\mu$m beträgt.

7. Hygieneprodukt nach Anspruch 1, in dem die Folie einen Stoff umfasst, der aus Fasern gebildet ist, deren Oberfläche durch ein hydrophobes polymeres Material bereitgestellt wird.

8. Hygieneprodukt nach Anspruch 7, in dem mindestens 40 Gew.-% des Materials der Fasern des Stoffes Polypropylen sind.

9. Hygieneprodukt nach Anspruch 7, in dem die mittlere Dicke der Fasern, aus denen der Stoff der oberen Folie gebildet ist, weniger als 30 μm beträgt.

10. Hygieneprodukt nach Anspruch 7, in dem die obere Folie ein Laminat umfasst, das aus einem ersten und einem zweiten nicht-gewebten Stoff gebildet ist.

11. Hygieneprodukt nach Anspruch 10, in dem der erste Stoff des Laminats aus Spinnfasern gebildet ist und der zweite Stoff ein schmelzgeblasener Stoff ist.

12. Hygieneprodukt nach Anspruch 10, in dem die mittlere Dicke der Fasern des zweiten Stoffes nicht mehr als 8 μm beträgt.

13. Hygieneprodukt nach Anspruch 10, in dem das Verhältnis des Gewichts der Fasern des zweiten Stoffes zum Gewicht der Fasern des gesamten Laminats mindestens 0,1 beträgt.

14. Hygieneprodukt nach Anspruch 10, in dem das Laminat einen dritten Stoff einschließt.

15. Verfahren zur Herstellung eines absorbierenden Hygieneprodukts, das einen Kern aus einem absorbierenden Material und eine obere Folie umfasst, durch welche zu absorbierendes Fluid zur Absorption durch das absorbierende Material tritt, welches umfasst:

   a. Imprägnieren einer porösen Folie mit Oberflächen, die durch ein hydrophobes polymeres Material bereitgestellt werden, mit einer Lösung eines Vinyl-Monomers, bei dem es sich um eine ethylenisch ungesättigte Säure oder ein Ester derselben handelt, so dass das Monomer hydrophile Eigenschaften zeigt oder abgeändert werden kann, dass es diese zeigt,
   b. Einwirkenlassen von Ultraviolettstrahlung auf die imprägnierte Folie, um zu bewirken, dass das Vinyl-Monomer mit dem Material der Folie copolymerisiert,
   c. Trocknen und Fertigstellen der Folie, um eine obere Folie zu produzieren, und
   d. Laminieren der oberen Folie mit einem Kern aus absorbierendem Material.

16. Verfahren nach Anspruch 15, in dem das Lösungsmittel für das Vinyl-Monomer während des Schritts der Einwirkung von Ultraviolettstrahlung auf die imprägnierte Folie nicht signifikant verdampft.

17. Verfahren nach Anspruch 15, in dem das Lösungsmittel für das Vinyl-Monomer mindestens 50 Gew.-% Wasser umfasst, bezogen auf das Gesamtgewicht der Lösung außer der bzw. den Vinyl-MonomerKomponente oder -Komponenten und jeglichen anderen signifikanten funktionellen Komponenten.

18. Verfahren nach Anspruch 15, indem die Lösung für das Vinyl-Monomer mindestens 5 Gew.-% eines organischen Lösungsmittels umfasst.

19. Verfahren nach Anspruch 15, welches den Schritt der mechanischen Behandlung der Folie einschließt, um die Imprägnierung der Folie durch die Vinyl-Monomer-Lösung zu fördern.

20. Verfahren nach Anspruch 15, in dem die Vinyl-Monomer-Lösung einen Initiator enthält, der eine atomare Spezies von dem Polymer der Folienoberfläche abstrahiert, so dass ein Polymer-Radikal auf der Folienoberfläche gebildet wird.

21. Verfahren nach Anspruch 15, in dem das Vinyl-Monomer eine Säure ist und das Verfahren den Schritt der zumindest teilweisen Neutralisation der Säure einschließt, um die Säure in ein Salz, bevorzugt ein Natriumsalz oder ein Kaliumsalz, zu überführen.

22. Absorbierendes Hygieneprodukt, das ein Kern aus einem absorbierenden Material und eine obere Folie umfasst, durch welche zu absorbierendes Fluid zur Absorption durch das absorbierende Material tritt, in dem die obere Folie porös ist und eine Folie mit Oberflächen umfasst, die von einem hydrophoben polymeren Material bereitgestellt werden und durch eine Pfropf-Copolymerisationsreaktion zwischen den Oberflächen und einem Vinyl-Monomer modifiziert worden sind, bei dem es sich um eine ethylenisch ungesättigte Säure oder einen Ester derselben handelt, so dass das Monomer hydrophile Eigenschaften zeigt oder abgeändert werden kann, dass es diese zeigt, und in dem das gepfropfte Vinyl-Monomer als im Wesentlichen gleichförmige Beschichtung auf den Folienoberflächen bereitgestellt wird, deren mittlere Dicke nicht mehr als 3,0 μm beträgt.

## EP 1 455 845 B1

**Revendications**

1. Produit d'hygiène absorbant qui comprend un coeur en un matériau absorbant et une feuille supérieure que le fluide qui doit être absorbé traverse pour être absorbé par le matériau absorbant, dans lequel la feuille supérieure est poreuse et comprend une feuille ayant des surfaces pourvues d'un matériau polymère hydrophobe qui ont été modifiées par une réaction de polymérisation par greffage entre les surfaces et un monomère de vinyle qui est un acide insaturé éthyléniquement ou un ester de celui-ci de façon à ce que le monomère présente ou puisse être modifié de manière à présenter des propriétés hydrophiles, et dans lequel le produit est fabriqué par un procédé qui implique l'exposition de la feuille à un rayonnement ultraviolet alors qu'elle est imprégnée avec une solution du monomère de vinyle.

2. Produit d'hygiène selon la revendication 1, qui contient des quantités à l'état de traces d'un initiateur pour la réaction de copolymération par greffage grâce auquel une espèce atomique a été ôtée du polymère de la surface de la feuille pour former un radical polymère sur la surface de la feuille.

3. Produit d'hygiène selon la revendication 1, dans lequel les groupes qui sont liés à la surface du matériau polymère hydrophobe comprennent un sel d'un acide insaturé éthyléniquement.

4. Produit d'hygiène selon la revendication 1, dans lequel le matériau polymère de la feuille comprend au moins une polyoléfine.

5. Produit d'hygiène selon la revendication 1, dans lequel le matériau polymère de la feuille comprend du polypropylène.

6. Produit d'hygiène selon la revendication 1, dans lequel l'épaisseur moyenne de la feuille supérieure est inférieure à 400 $\mu$m.

7. Produit d'hygiène selon la revendication 1, dans lequel la feuille comprend un tissu formé à partir de fibres dont la surface est pourvue d'un matériau polymère hydrophobe.

8. Produit d'hygiène selon la revendication 7, dans lequel au moins 40 % en poids du matériau de fibres du tissu est du polypropylène.

9. Produit d'hygiène selon la revendication 7, dans lequel l'épaisseur moyenne des fibres à partir desquelles le tissu de la feuille supérieure est formé est inférieure à 30 $\mu$m.

10. Produit d'hygiène selon la revendication 7, dans lequel la feuille supérieure comprend un stratifié formé à partir d'un premier et d'un second tissus non tissés.

11. Produit d'hygiène selon la revendication 10, dans lequel le premier tissu du stratifié est formé à partir de fibres filées et le second tissu est un tissu obtenu par soufflage à chaud.

12. Produit d'hygiène selon la revendication 10, dans lequel l'épaisseur moyenne des fibres du second tissu ne dépasse pas 8 $\mu$m.

13. Produit d'hygiène selon la revendication 10, dans lequel le rapport entre le poids des fibres du second tissu et le poids des fibres du stratifié entier est d'au moins 0,1.

14. Produit d'hygiène selon la revendication 10, dans lequel le stratifié comprend un troisième tissu.

15. Procédé de fabrication d'un produit d'hygiène absorbant comprenant un coeur en un matériau absorbant et une feuille supérieure que le fluide qui doit être absorbé traverse pour être absorbé par le matériau absorbant, qui comprend les étapes consistant à :

   a. imprégner une feuille poreuse, ayant des surfaces qui sont pourvues d'un matériau polymère hydrophobe, avec une solution d'un monomère de vinyle qui est un acide insaturé éthyléniquement ou un ester de celui-ci de façon à ce que le monomère présente ou puisse être modifié de manière à présenter des propriétés hydrophiles,
   b. exposer la feuille imprégnée à un rayonnement ultraviolet pour induire la copolymérisation du monomère de vinyle avec le matériau de la feuille,
c. sécher et appliquer un fini à la feuille pour produire une feuille supérieure, et
d. stratifier la feuille supérieure avec un coeur en matériau absorbant.

**16.** Procédé selon la revendication 15, dans lequel le solvant pour le monomère de vinyle ne s'évapore pas de manière significative pendant l'étape d'exposition de la feuille imprégnée au rayonnement ultraviolet.

**17.** Procédé selon la revendication 15, dans lequel le solvant pour le monomère de vinyle comprend au moins 50 % en poids d'eau, sur la base du poids total de la solution hors composant(s) de monomère de vinyle, et de tout autre composant fonctionnel significatif.

**18.** Procédé selon la revendication 15, dans lequel la solution pour le monomère de vinyle comprend au moins 5 % en poids d'un solvant organique.

**19.** Procédé selon la revendication 15, qui comprend l'étape consistant à traiter la feuille mécaniquement pour favoriser l'imprégnation de la feuille par la solution de monomère de vinyle.

**20.** Procédé selon la revendication 15, dans lequel la solution de monomère de vinyle contient un initiateur qui ôte une espèce atomique du polymère de la surface de la feuille pour former un radical polymère sur la surface de la feuille.

**21.** Procédé selon la revendication 15, dans lequel le monomère de vinyle est un acide, et le procédé comprend l'étape consistant à neutraliser l'acide, au moins partiellement, pour convertir l'acide en un sel, de préférence, un sel de sodium ou un sel de potassium.

**22.** Produit d'hygiène absorbant qui comprend un coeur en un matériau absorbant et une feuille supérieure que le fluide qui doit être absorbé traverse pour être absorbé par le matériau absorbant, dans lequel la feuille supérieure est poreuse et comprend une feuille ayant des surfaces pourvues d'un matériau polymère hydrophobe qui ont été modifiées par une réaction de polymérisation par greffage entre les surfaces et un monomère de vinyle qui est un acide insaturé éthyléniquement ou un ester de celui-ci de façon à ce que le monomère présente ou puisse être modifié de manière à présenter des propriétés hydrophiles, et dans lequel le monomère de vinyle greffé est fourni sous la forme d'un revêtement essentiellement uniforme sur les surfaces de la feuille dont l'épaisseur moyenne ne dépasse pas 3,0 $\mu$m.

**Figure 1**

Figure 2b

Figure 2a